# EUROPEAN PATENT APPLICATION

(11) **EP 4 109 132 A1**
(43) Date of publication of application: **28.12.2022**
(21) Application number: 22180180.6
(22) Date of filing: 21.06.2022
(51) Int. Cl.: G01S 7/52, G01S 15/89, G10K 11/34

(54) **ULTRASOUND DIAGNOSTIC APPARATUS AND EXTRACTION METHOD**

(30) Priority: 21.06.2021 JP 2021102206
(71) Applicant: Canon Medical Systems Corporation, Tochigi 324-0036 (JP)
(72) Inventor: SATO, Takeshi, Tochigi (JP)
(74) Representative: Marks & Clerk LLP

(57) **Abstract**

An ultrasound diagnostic apparatus according to an embodiment includes a transmitting unit (101), a beam-forming processing unit (120), and an extracting unit (130). The transmitting unit (101) performs transmission of ultrasonic pulses of different polarities to different transmission positions of a subject for multiple times. The beam-forming processing unit (120) performs, for each of the different polarities, reception beam-forming processing with respect to plural reception signals acquired by transmission of plural ultrasonic pulses of an identical polarity. The extracting unit (130) extracts a non-linear signal by adding up reception signals of the different polarities at an identical reception position, the signals subjected to reception beam forming.

## Description

### FIELD

Embodiments described herein relate generally to an ultrasound diagnostic apparatus and an extraction method.

### BACKGROUND

In ultrasound diagnostic apparatuses, an imaging technique using non-linear phenomenon of ultrasound propagation in a living body called tissue harmonic imaging (THI) has become a mainstream in recent years as an imaging technique used when B-mode image data is generated. Moreover, contrast harmonic imaging (CHI) of rendering a blood vessel by using non-linear phenomenon of ultrasound contrast agent injected into a blood vessel has also been used. In these THI and CHI, pulse inversion method is common as a method of acquiring a non-linear signal. In the pulse inversion method, by transmitting a first ultrasonic pulse starting from the positive polarity (positive-going first ultrasonic pulse) and a second ultrasonic pulse starting from the negative polarity (negative-going second ultrasonic pulse) that is a pulse, the polarity of which is an inverted polarity of the first ultrasonic pulse, to the same position, and by adding up two reception signals acquired by transmission of these two ultrasonic pulses, a non-linear component is extracted, suppressing a fundamental wave component. However, to transmit ultrasonic pulses to the same position twice, the frame rate decreases to half (1/2) compared to a case in which an ultrasonic pulse is transmitted to the same position once. If the number of parallel simultaneous reception is increased by decreasing the density of transmission scan line (transmission raster) to increase the frame rate, a problem occurs that streaks appear in an ultrasonic image. A reason why such a problem occurs is because the spatial sampling theorem is not satisfied when the density of transmission scan line is small, for example, because an azimuth resolution is high, that is, a spatial frequency is high, near a transmission focus (transmission focal point).

A method of adding up two reception signals of the same reception position (same position) acquired by two-way parallel simultaneous reception by transmitting the first ultrasonic pulse and the second ultrasonic pulse not to the same position, but to different positions, has been known. The problem that the frame rate decreases described above can be solved by this method. It is preferable that a component that is an inverted fundamental wave component included in one reception signal out of the two reception signals of the same position and a fundamental wave component included in the other reception signal coincide with each other. This is because the fundamental wave component is suppressed by adding the other reception signal to one reception signal. However, because the position to which the positive-going ultrasonic pulse is transmitted and the position to which the negative-going ultrasonic pulse are different from each other, there is a case in which a component that is an inverted fundamental wave component included in one reception signal and a fundamental wave component included in the other reception signal do not coincide with each other. In this case, the fundamental wave component remains.

Accordingly, not only a non-linear signal, but also a linear signal is extracted by addition, and it is not preferable for THI and CHI. That is, there is a problem that an image quality is not preferable.

A technique of applying transmission focus to a point other than a transmission focus by a technique of transmission aperture synthesis using a virtual sound source, by using plural pieces of reception data acquired by plural ultrasonic pulses transmitted to positions different from one another has been known.

### Summary of Invention

An ultrasound diagnostic apparatus provided in one aspect of an embodiment includes a transmitting unit, a beam-forming processing unit and an extracting unit. The transmitting unit performs transmission of ultrasonic pulses of different polarities to different transmission positions of a subject for multiple times. The beam-forming processing unit performs, for each of the different polarities, reception beam-forming processing with respect to plural reception signals acquired by transmission of plural ultrasonic pulses of an identical polarity. The extracting unit extracts a non-linear signal by adding up reception signals of the different polarities at an identical reception position, the signals subjected to reception beam forming.

The beam-forming processing unit may correct a misalignment caused by transmission positions being different, the transmission positions to which the ultrasonic waves of different polarities are transmitted, in the reception beam-forming processing.

The beam-forming processing unit may correct the misalignment by a virtual sound source method by using the reception signals acquired by transmission of the ultrasonic pulses.

The beam-forming processing unit may correct the misalignment that is acquired by measurement or simulation by using the reception signals acquired by transmission of the ultrasonic pulses.

The beam-forming processing unit may perform, for each of the different polarities, the reception beam-forming processing with respect to the plurality of the reception signals at once.

The transmitting unit may perform the transmission of the ultrasonic pulses of two polarities that differ from each other to the transmission positions for the plurality of times,
the beam-forming processing unit may perform, for each of two polarities, the reception beam-forming processing with respect to the plurality of the reception signals, and
the extracting unit may extract the non-linear signal by adding up the reception signals of two polarities at the identical reception position, the reception signals subjected to the reception beam forming.

An extraction method provided in one aspect of an embodiment includes performing transmission of ultrasonic pulses of different polarities to different transmission positions of a subject for multiple times, performing, for each of the different polarities, reception beam-forming processing with respect to plural reception signals acquired by transmission of plural ultrasonic pulses of an identical polarity, and extracting a non-linear signal by adding up reception signals of the different polarities at an identical reception position, the signals subjected to reception beam forming.

The performing the reception beam-forming processing may include correcting a misalignment caused by transmission positions being different, the transmission positions to which the ultrasonic waves of different polarities are transmitted, by the reception beam-forming processing.

The performing the reception beam-forming processing may include correcting the misalignment by a virtual sound source method by using the reception signals acquired by transmission of the ultrasonic pulses.

The performing the reception beam-forming processing may include correcting the misalignment that is acquired by any one of measurement and simulation by using the reception signals acquired by transmission of the ultrasonic pulses.

The performing the reception beam-forming processing may include performing, for each of the different polarities, the reception beam-forming processing with respect to the plural reception signals at once.

The performing the transmission of the ultrasonic pulses may include performing the transmission of the ultrasonic pulses of two polarities that differ from each other to the transmission positions for the multiple times. The performing the reception beam-forming processing may include performing, for each of two polarities, the reception beam-forming processing with respect to the plural reception signals. The extracting the non-linear signal may include extracting the non-linear signal by adding up the reception signals of two polarities at the identical reception position, the reception signals subjected to the reception beam forming.

### BRIEF DESCRIPTION OF THE DRAWINGS

FIG. 1 is a block diagram illustrating a configuration example of an ultrasound diagnostic apparatus according to a first embodiment;
FIG. 2 is a diagram for explaining one example of processing performed by a conventional ultrasound diagnostic apparatus;
FIG. 3 is a diagram for explaining one example of processing performed by the ultrasound diagnostic apparatus according to the first embodiment;
FIG. 4 is a diagram for explaining one example of a problem of a conventional ultrasound diagnostic apparatus;
FIG. 5 is an enlarged view of an area in FIG. 4; and
FIG. 6 is a flowchart illustrating a flow of one example of processing performed by the ultrasound diagnostic apparatus according to the first embodiment.

### DETAILED DESCRIPTION

A challenge of embodiments disclosed in the present application and the drawings are to improve an image quality while suppressing reduction of a frame rate. However, challenges of the embodiments disclosed in the present application and the drawings are not limited to the above challenge. Challenges corresponding to respective effects by respective components described in embodiments described later can be regarded as other challenges.

An ultrasound diagnostic apparatus of the embodiment includes a transmitting unit, a beam-forming processing unit, and an extracting unit. The transmitting unit performs transmission of ultrasonic pulses of different polarities to different transmission positions of a subject multiple times. The beam-forming processing unit performs, for each of the different polarities, reception beam forming with respect to plural reception signals that are acquired by transmission of plural ultrasonic pulses of the same polarity. The extracting unit extracts a non-linear signal by adding up reception signals of the different polarities at the same reception position after the reception beam forming.

Hereinafter, the ultrasound diagnostic apparatus according to the embodiment will be explained with reference to the drawings. The embodiment can be combined with a conventional technique, other embodiments, or other modifications within a range not causing contradictions. Similarly, a modification can be combined with a conventional technique, other embodiments, or other modifications within a range not causing contradictions. Moreover, in the following explanation, common reference symbols are given to like components, and duplicated explanation may be omitted. First Embodiment

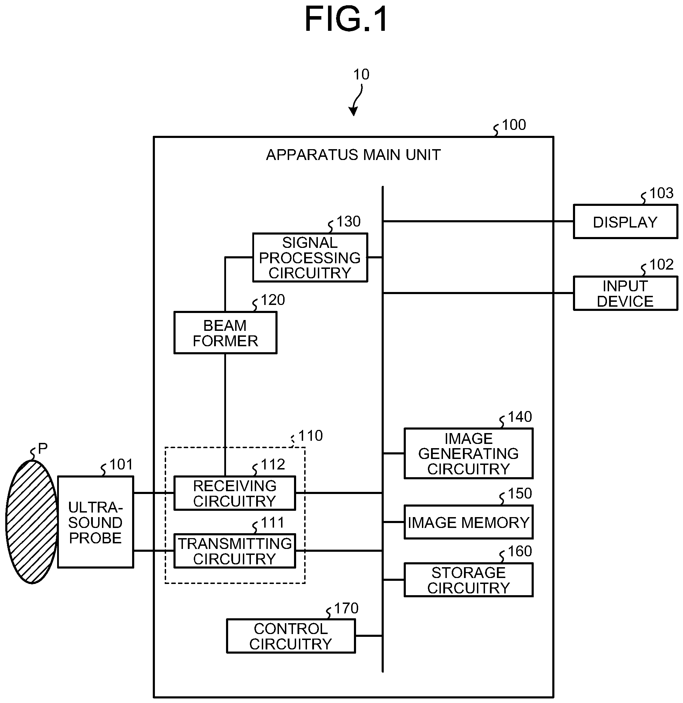

FIG. 1 is a block diagram illustrating a configuration example of an ultrasound diagnostic apparatus 10 according to a first embodiment. As illustrated in FIG. 1, the ultrasound diagnostic apparatus 10 according to the first embodiment includes an apparatus main unit 100, an ultrasound probe 101, an input device 102, and a display 103.

The ultrasound probe 101 includes, for example, plural transducers (piezoelectric devices). These plural transducers generate ultrasonic waves based on a driving signal provided by transmitting circuitry 111 of a transceiving circuitry 110 included in the apparatus main unit 100. Specifically, the transducers generate an ultrasonic wave having a waveform according to a transmission driving voltage, being applied with a voltage (transmission driving voltage) by the transmitting circuitry 111. Furthermore, the ultrasound probe 101 receives a reflected wave from a subject P, converts the reflected wave into a reflection wave signal, which is an electric signal, and outputs (transmits) the reflection wave signal to the apparatus main unit 100. The reflection wave signal is one example of a reception signal. Moreover, the ultrasound probe 101 includes, for example, a matching layer that is arranged in the transducer, backing material to prevent propagation of an ultrasonic wave to a rearward direction from the transducer, and the like. The ultrasound probe 101 is detachably connected to the apparatus main unit 100.

When an ultrasonic wave (transmission ultrasonic wave, ultrasonic pulse) is transmitted to the subject P from the ultrasound probe 101, the transmitted ultrasonic wave is sequentially reflected on a discontinuous surface of an acoustic impedance in an internal tissue of the subject P, and is received by the transducers included in the ultrasound probe 101 as reflected waves. An amplitude of the received reflected wave is dependent on a difference in acoustic impedance on the discontinuous surface on which the ultrasonic wave is reflected. A reflected wave that is the transmitted ultrasonic pulse reflected on a surface of flowing blood, a heart wall, or the like is subjected to frequency shift depending on a speed component with respect to an ultrasonic wave transmission direction of a moving body by the Doppler effect. The ultrasound probe 101 transmits the reflected wave to receiving circuitry 112 of the transceiving circuitry 110 described later.

The ultrasound probe 101 is arranged detachably to the apparatus main unit 100. When scanning (two-dimensional scanning) of a two-dimensional area in the subject P is performed, an operator connects, for example, 1D array probe in which plural transducers are arranged in one row to the apparatus main unit as the ultrasound probe 101. Types of the 1D array probe include a linear ultrasound probe, a convex ultrasound probe, a sector ultrasound probe, and the like. Moreover, when scanning (three-dimensional scanning) of a three-dimensional area in the subject P, the operator connects, for example, a mechanical 4D probe or 2D array probe to the apparatus main unit 100 as the ultrasound probe 101. The mechanical 4D probe is capable of two-dimensional scanning by using plural transducers arranged in one row as in the 1D array probe, and is also capable of three-dimensional scanning by swinging the transducers at a predetermined angle (swinging angle). Furthermore, the 2D array probe is capable of three-dimensional scanning by plural transducers arranged in a matrix form, and is also capable of two-dimensional scanning by transmitting ultrasonic waves in a focused manner.

The input device 102 is implemented by, for example, an input means, such as a mouse, a keyboard, a button, a panel switch, a touch command screen, a foot switch, a trackball, and a joystick. The input device 102 accepts various kinds of setting request from an operator of the ultrasound diagnostic apparatus 10, and transfers the accepted various kinds of setting request to the apparatus main unit 100.

The display 103 displays a graphical user interface (GUI) for an operator of the ultrasound diagnostic apparatus 10 to input various kinds of setting requests by using the input device 102, or displays a ultrasonic image based on ultrasonic image data generated in the apparatus main unit 100, and the like. The display 103 is implemented by a liquid crystal monitor, a cathode ray tube monitor, or the like.

The apparatus main unit 100 generates ultrasonic image data based on a reflected wave signal transmitted from the ultrasound probe 101. The ultrasonic image data is one example of image data. The apparatus main unit 100 can generate two-dimensional ultrasonic image data based on a reflected wave signal corresponding to a two-dimensional area of the subject P transmitted from the ultrasound probe 101. Moreover, the apparatus main unit 100 can generate three-dimensional ultrasonic image data based on a reflected wave signal corresponding to a three-dimensional area of the subject P transmitted from the ultrasound probe 101. As illustrated in FIG. 1, the apparatus main unit 100 includes transceiving circuitry 110, a beam former 120, signal processing circuitry 130, image generating circuitry 140, an image memory 150, storage circuitry 160, and control circuitry 170.

The transceiving circuitry 110 causes the ultrasound probe 101 to transmit an ultrasonic wave, and causes the ultrasound probe 101 to receive a reflected wave of the ultrasonic wave, in response to a control of the control circuitry 170. That is, the transceiving circuitry 110 performs scanning through the ultrasound probe 101. The scanning is also called scan, ultrasonic scan, or ultrasonic scanning. The transceiving circuitry 110 is one example of a transceiving unit. The transceiving circuitry 110 includes the transmitting circuitry 111 and the receiving circuitry 112.

The transmitting circuitry 111 supplies a driving signal to the ultrasound probe 101, and causes the ultrasound probe 101 to transmit an ultrasonic wave in response to a control by the control circuitry 170. The transmitting circuitry 111 includes rate-pulser generating circuitry, transmission delaying circuitry, and a transmission pulser. When a two-dimensional area inside the subject P is to be scanned, the transmitting circuitry 111 causes the ultrasound probe 101 to transmit an ultrasonic beam to scan the two-dimensional area. Moreover, when a three-dimensional area inside the subject P is to be scanned, the transmitting circuitry 111 causes the ultrasound probe 101 to transmit an ultrasonic beam to scan the three-dimensional area.

The rate-pulser generating circuitry repeatedly generates a rate pulse to form a transmission ultrasonic wave (transmission beam) at a predetermined rate frequency (pulse repetition frequency (PRF)) in response to a control by the control circuitry 170. As the rate pulse passing through the transmission delaying circuitry, a voltage is applied to the transmission pulser in a state of having different transmission delay time. For example, the transmission delaying circuitry gives transmission delay time for each transducer necessary to determine transmission directivity to each rate pulse generated by the rate-pulse generating circuitry by focusing ultrasonic waves generated by the ultrasound probe 101 into a beam form. The transmission pulser supplies a driving signal (driving pulse) to the ultrasound probe 101 in timing based on a rate pulse. The transmission delaying circuitry adjusts a transmission direction of an ultrasonic wave from a transducer surface by changing the transmission delay time to be applied to each rate pulse.

After the driving pulse reaches the transducer in the ultrasound probe 101 through a cable from the transmission pulser, it is converted from an electrical signal to mechanical vibration in the transducer. That is, as a voltage is applied to the transducer, the transducer mechanically vibrates. An ultrasonic wave generated by this mechanical vibration is transmitted to the inside of a living body. The ultrasonic wave having transmission delay time different in each transducer is focused and is propagated into a predetermined direction.

The transmitting circuitry 111 has a function of instantly changing transmission frequency, transmission driving voltage, and the like to perform a predetermined scanning sequence in response to a control by the control circuitry 170. Particularly, change of the transmission driving voltage is implemented by linear amplifier type transmitting circuitry that can change a value of a transmission driving voltage instantly, or a mechanism that electrically switches among plural power source units.

After reaching the transducer in the ultrasound probe 101, a reflected wave of the ultrasonic wave transmitted by the ultrasound probe 101 is converted into electrical signal (reflection wave signal) from the mechanical vibration in the transducer, and the converted reflection wave signal is input to the receiving circuitry 112. That is, an analog reflection wave signal is input to the receiving circuitry 112. The receiving circuitry 112 includes a low noise amplifier (LNA), analog time gain compensation (ATGC) processing circuitry, an analog to digital convertor, a demodulator, and the like, and subjects the reflection wave signal transmitted from the ultrasound probe 101 to various kinds of processing, to generate an in-phase signal (I signal) of a baseband bandwidth and a quadrature-phase signal (Q signal) as a digital reflection wave signal. The I signal and the Q signal are called IQ signal. The receiving circuitry 112 transmits the generated IQ signal to the beam former 120 as a reflection wave signal (reception signal).

The transmitting circuitry 111 causes the ultrasound probe 101 to transmit an ultrasonic beam from a predetermined transmission position (transmission scan line). The receiving circuitry 112 receives a reflection wave signal by the reflected wave of the ultrasonic beam transmitted by the transmitting circuitry 111 at a predetermined reception position (reception scan line) from the ultrasound probe 101. When parallel simultaneous reception is not performed, the transmission scan line and the reception scan line are the same scan line. In contrast, in the case of performing parallel simultaneous reception, when the transmitting circuitry 111 causes the ultrasound probe 101 to transmit one ultrasonic beam at one transmission scan line, the receiving circuitry 112 receives a reflection wave signal by a reflected wave originating from the ultrasonic beam, which has been transmitted by the ultrasound probe 101 caused to transmit it by the transmitting circuitry 111, as plural reception beams at plural predetermined reception positions (reception scan lines) simultaneously through the ultrasound probe 101.

The beam former 120 performs reception phasing addition (reception beam forming) and transmission phasing addition (transmission beam forming) with respect to the reflection wave signal transmitted by the receiving circuitry 112, and thereby generates reflection wave data. As described above, although the reflection wave signal is one example of a reception signal, the reflection wave data is also one example of a reception signal. The beam former 120 transmits the generated reflection wave data to the signal processing circuitry 130. The beam former 120 is implemented by, for example, a processor. Details of the beam former 120 will be described later.

The signal processing circuitry 130 receives the reflection wave signal transmitted by the beam former 120, subjects the received reflection wave signal to various kinds of signal processing, and transmits the reflection wave data subjected to the various kinds of signal processing to the image generating circuitry 140 as B-mode data or doppler data. The signal processing circuitry 130 is implemented by, for example, a processor. The signal processing circuitry 130 is one example of a signal processing unit. Hereinafter, one example of various kinds of signal processing performed by the signal processing circuitry 130 will be explained.

For example, the signal processing circuitry 130 performs various kinds of processing, such as envelope detection processing and logarithmic compression processing, with respect to the reflection wave data, and generates B-mode data in which a signal intensity (amplitude intensity) of each sample point is expressed by brightness. For example, the signal processing circuitry 130 includes an envelope detector, a logarithmic compressor, and the like. For example, the envelope detector performs envelope detection of the reflection wave data, and the logarithmic compressor logarithmic-compresses data relating to an envelope acquired by the envelope detection (for example, data indicating an amplitude, or the like). Thus, B-mode data is generated. The signal processing circuitry 130 transmits the generated B-mode data to the image generating circuitry 140.

Moreover, the signal processing circuitry 130 performs signal processing to perform harmonic imaging to visualize a harmonic component. The harmonic imaging includes CHI and THI. Furthermore, in CHI or THI, for example, phase modulation (PM) called pulse inversion is known as a scanning method.

Moreover, the signal processing circuitry 130 performs frequency analysis of the reflection wave data, to thereby extract movement information of a moving object (blood, tissue, echo component of contrast agent, and the like) based on the Doppler effect from the reflection wave data, and generates doppler data indicating the extracted movement information. For example, the signal processing circuitry 130 extracts a mean speed, a mean variance value, a mean power value, and the like as the movement information of a moving object from multiple points, and generates doppler data indicating the extracted movement information of the moving body. The signal processing circuitry 130 transmits the generated doppler data to the image generating circuitry 140.

By using the function of the signal processing circuitry 130 described above, the ultrasound diagnostic apparatus 10 according to the embodiment is capable of performing a color doppler technique also called color flow mapping (CFM). In the color flow mapping, transmission and reception of ultrasonic waves are performed plural times on plural scan lines. In the color flow mapping, by applying a moving target indicator (MTI) filter to a data string of the same position, a signal originating from blood flow (blood flow signal) is extracted from the data string of the same position, suppressing a signal originating from a still tissue, or tissue moving slowly (clutter signal). In the color flow mapping, blood flow information, such as speed of blood flow, variance of blood flow, and power of blood flow, is estimated from this blood flow signal. The signal processing circuitry 130 transmits color image data indicating the blood flow information estimated by the color flow mapping to the image generating circuitry 140. The color image data is one example of the doppler data.

The signal processing circuitry 130 can process both of reflection wave data, two-dimensional reflection wave data and three-dimensional reflection wave data.

The image generating circuitry 140 generates ultrasound image data from the B-mode data or the doppler data transmitted by the signal processing circuitry 130. The image generating circuitry 140 is implemented by a processor.

For example, the image generating circuitry 140 generates two-dimensional B-mode image data in which the intensity of a reflected wave is expressed by brightness from the two-dimensional B-mode data generated by the signal processing circuitry 130. Moreover, the image generating circuitry 140 generates two-dimensional doppler image data in which movement information or blood flow information is visualized from the two-dimensional doppler data generated by the signal processing circuitry 130. The two-dimensional doppler image data in which movement information is visualized is speed image data, variance image data, power image data, or image data in which these are combined.

Generally, the image generating circuitry 140 converts a scan-line signal string of ultrasound scanning into a scan-line signal string of a video format typified by television or the like (scan conversion), to generate ultrasonic image data for display. For example, the image generating circuitry 140 generates ultrasonic image data for display by performing coordinate conversion according to a scan mode of a ultrasonic wave by the ultrasound probe 101 with respect to data transmitted by the signal processing circuitry 130. Moreover, the image generating circuitry 140 performs, as various image processing other than the scan conversion, for example, image processing of reproducing a mean image of brightness (smoothing processing), image processing using a differential filter in an image (edge reinforcement processing), and the like by using plural image frames after the scan conversion. Furthermore, the image generating circuitry 140 adds textual information of various parameters, scale marks, body mark, and the like to the ultrasound image data.

Moreover, the image generating circuitry 140 generates three-dimensional B-mode image data by performing coordinate conversion with respect to three-dimensional B-mode data generated by the signal processing circuitry 130. Furthermore, the image generating circuitry 140 generates three-dimensional doppler image data by performing coordinate conversion with respect to three-dimensional doppler data generated by the signal processing circuitry 130. That is, the image generating circuitry 140 generates "three-dimensional B-mode image data and three-dimensional doppler image data" as "three-dimensional ultrasound data (volume data)". The image generating circuitry 140 performs various rendering processing with respect to the volume data, to generate various kinds of two-dimensional image data to display the volume data on the display 103.

The rendering processing performed by the image generating circuitry 140 includes, for example, processing of generating multi planer reconstruction (MPR) image data from the volume data by using MPR. Moreover, the rendering processing performed by the image generating circuitry 140 includes, for example, volume rendering (VR) processing to generate two-dimensional image data in which three-dimensional information is reflected. The image generating circuitry 140 is one example of an image generating unit.

The B-mode data and the doppler data are ultrasound image data before being subjected to the scan conversion processing, and data generated by the image generating circuitry 140 is ultrasound image data for display after being subjected to the scan conversion processing. The B-mod data and the doppler data are also called raw data.

The image memory 150 is a memory that stores various kinds of image data generated by the image generating circuitry 140. Moreover, the image memory 150 also stores data generated by the signal processing circuitry 130. The B-mode data and the doppler data stored in the image memory 150 can be called by an operator after diagnosis, for example, and become ultrasound image data for display through the image generating circuitry 140. For example, the image memory 150 is implemented by a semiconductor memory device, such as a random access memory (RAM) and a flash memory, a hard disk, or an optical disk.

The storage circuitry 160 stores a control program to perform scanning (transmission and reception of an ultrasonic wave), image processing, and display processing, or various kinds of data, such as diagnostic information (for example, patient ID, findings of doctor, and the like), a diagnostic protocol, and various kinds of body marks. Furthermore, the storage circuitry 160 is also used for storing data stored in the image memory 150 as necessary. For example, the storage circuitry 160 is implemented by a semiconductor memory device, such as a flash memory, a hard disk, or an optical disk.

The control circuitry 170 overall controls processing of the ultrasound diagnostic apparatus 10. Specifically, the control circuitry 170 controls processing of the transceiving circuitry 110, the beam former 120, the signal processing circuitry 130, and the image generating circuitry 140 based on various kinds of setting requests input by an operator through the input device 102 or various kinds of control program and various kinds of data read from the storage circuitry 160. Moreover, the control circuitry 170 controls the display 103 to display an ultrasonic image based on the ultrasonic image data for display stored in the image memory 150. For example, the control circuitry 170 controls the display 103 to display a B-mode image based on the B-mode image data, or a color image based on the color image data. Furthermore, the control circuitry 170 controls the display 103 to display the B-mode image, superimposing a color image thereon. The control circuitry 170 is one example of a display control unit or a control unit. The control circuitry 170 is implemented by, for example, a processor. The ultrasonic image is one example of an image.

Moreover, the control circuitry 170 performs control of ultrasonic scanning by controlling the ultrasound probe 101 through the transceiving circuitry 110.

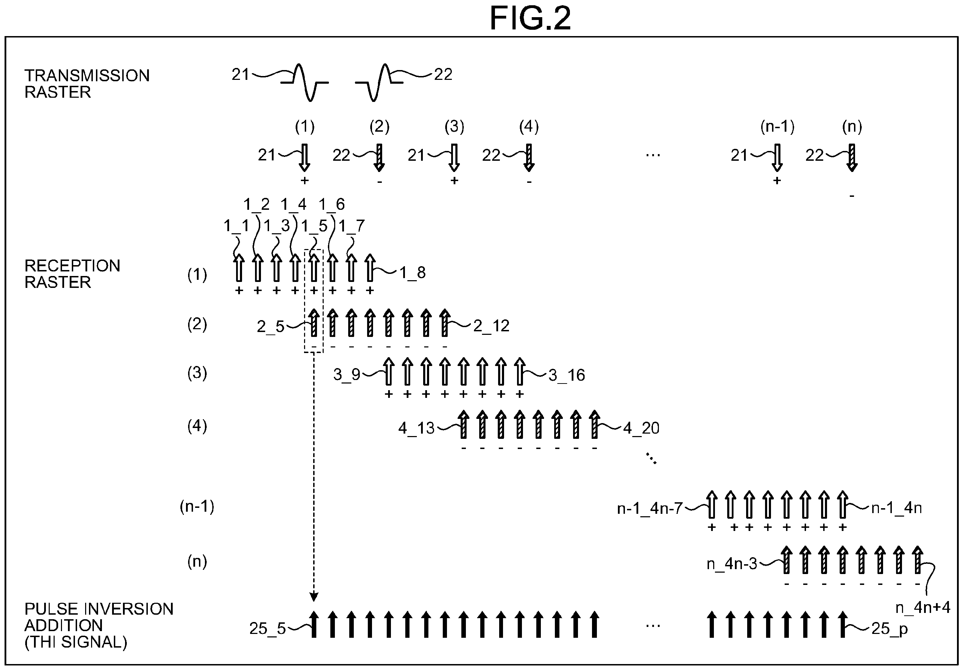

As above, an entire configuration of the ultrasound diagnostic apparatus 10 according to the first embodiment has been explained. An example of processing performed by a conventional ultrasound diagnostic apparatus will be explained, referring to FIG. 2. FIG. 2 is a diagram for explaining one example of processing performed by a conventional ultrasound diagnostic apparatus.

As illustrated in FIG. 2, a conventional ultrasound diagnostic apparatus transmits a first ultrasonic pulse starting from the positive polarity (transmission ultrasonic wave) 21 and a second ultrasonic pulse starting from the negative polarity that is a pulse in which the polarity of the first ultrasonic pulse is inverted to different transmission positions (transmission scan line). The conventional ultrasound diagnostic apparatus adds up signals at the same reception position acquired by parallel simultaneous reception. Hereinafter, it will be explained with a specific example.

For example, as illustrated in FIG. 2, the conventional ultrasound diagnostic apparatus transmits the first ultrasonic pulse 21 to each of a transmission scan line (1), a transmission scan line (3), ..., a transmission scan line (n-3), and a transmission scan line (n-1). Note that "n" is a positive integer, and is an even number. Furthermore, the conventional ultrasound diagnostic apparatus transmits the second ultrasonic pulse 22 to each of a transmission scan line (2), a transmission scan line (4), ..., a transmission scan line (n-2), and a transmission scan line (n).

The conventional ultrasound diagnostic apparatus generates, for example, as illustrated in FIG. 2, reflection wave data of plural reception positions (reception scan lines) as reflection wave data based on a reflected waved originating from the first ultrasonic pulse 21 transmitted to the transmission scan line (1). Specifically, the conventional ultrasound diagnostic apparatus generates eight pieces of reflection wave data 1_1 to 1_8 of eight reception scan lines (1) to (8), as reflection wave data based on a reflected wave originating from the first ultrasonic pulse 21 transmitted to the transmission scan line (1). Reflection wave data d_k (d, k are positive integers) is reflection wave data of a reception scan line (k) based on a reflected wave originating from the ultrasonic pulse transmitted to the transmission scan line (d). Note that "d" and "k" are positive integers.

The conventional ultrasound diagnostic apparatus generates eight pieces of reflection wave data d_k based on a reflected wave originating from the first ultrasonic pulse 21 similarly for each of other transmission scan lines (3), ..., (n-1) also. That is, the conventional ultrasound diagnostic apparatus generates eight pieces of reflection wave data s_(4s-3) to s_(4s+4) as reflection data based on a reflected wave originating from the first ultrasonic pulse 21 transmitted to the transmission scan line (s). Note that "s" is a positive integer, and is an odd number.

Moreover, the conventional ultrasound diagnostic apparatus generates, for example, as illustrated in FIG. 2, reflection wave data of plural reception positions (reception scan lines) as reception wave data based on a reflected wave originating from the second ultrasonic pulse 22 transmitted to the transmission scan line (2). Specifically, the conventional ultrasound diagnostic apparatus generates eight pieces of reflection wave data 2_5 to 2_12 of eight reception scan lines (5) to (12), as reflection wave data based on a reflected wave originating from the second ultrasonic pulse 22 transmitted to the transmission scan line (2).

The conventional ultrasound diagnostic apparatus generates eight pieces of reflection wave data d_k based on a reflected wave originating from the second ultrasonic pulse 22 similarly for each of other transmission scan lines (4), ..., (n) also. That is, the conventional ultrasound diagnostic apparatus generates eight pieces of reflection wave data w_(4w-3) to w_(4w+4) of eight reception scan lines (4w-3) to (4w+4) as reflection data based on a reflected wave originating from the second ultrasonic pulse 22 transmitted to the transmission scan line (w). Note that "w" is a positive integer, and is an even number.

The conventional ultrasound diagnostic apparatus then adds up the two pieces of the reflection wave data s_h, w_h of the same reception position. That is, the conventional ultrasound diagnostic apparatus adds the reflection wave data w_h to the reception wave data s_h, to generate reflection wave data 25_h (THI signal 25_h) that is a non-linear signal in which a fundamental wave component is suppressed. Note that "h" is a positive integer. For example, the conventional ultrasound diagnostic apparatus adds the reflection wave data 2_5 to the reflection wave data 1_5, to generate reflection wave data 25_5. As described, the conventional ultrasound diagnostic apparatus generates reflection wave data 25_h each time two pieces of the reflection wave data s_h, w_h are generated in one reception position. Specifically, the conventional ultrasound diagnostic apparatus generates (4n-4) pieces of reflection wave data 25_5 to 25_p. Note that "p" is 4n.

It is preferable that out of two pieces of the reflection wave data s_h, w_h of the same position, a component in which a fundamental wave component included in one of the reflection wave data s_h is inverted and a fundamental wave component included in the other one of the reflection wave data w_h coincide with each other. This is because the fundamental wave component is suppressed by adding the other one of the reflection wave data w_h to the one of the reflection wave data s_h. However, because the position to which the positive-going ultrasonic pulse is transmitted and the position to which the negative-going ultrasonic pulse is transmitted differ, there is a case in which a component that is an inverted fundamental component included in the reflection wave data s_h and a fundamental wave component included in the other reflection wave data w_h do not coincide with each other. In this case, the fundamental wave component remains.

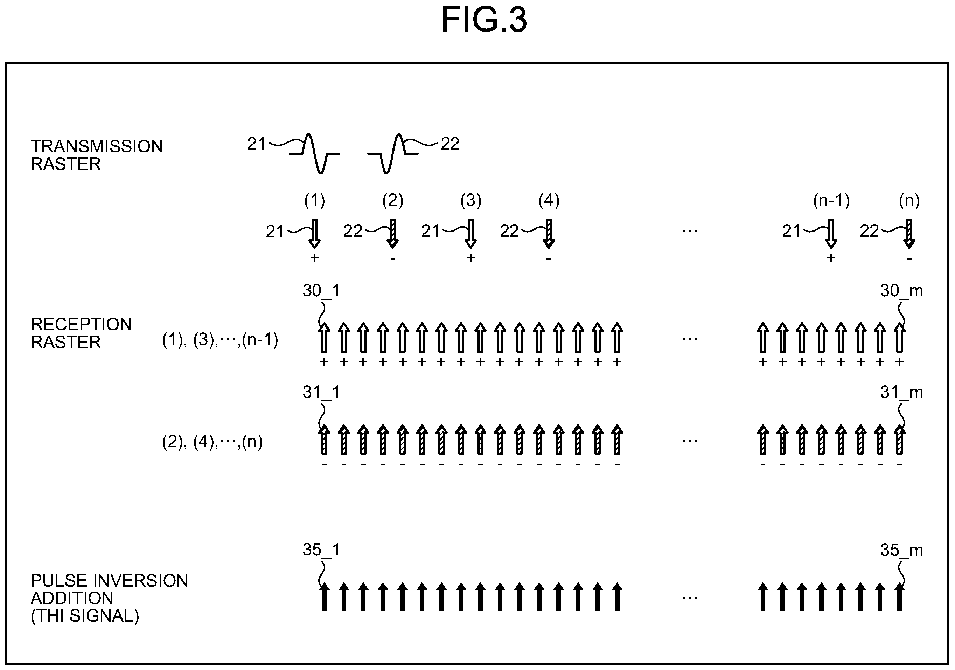

To prevent a fundamental wave component from remaining in the THI signal, the ultrasound diagnostic apparatus 10 according to the first embodiment performs processing explained below. FIG. 3 is a diagram for explaining one example of processing performed by the ultrasound diagnostic apparatus 10 according to the first embodiment. As illustrated in FIG. 3, the ultrasound probe 101 of the ultrasound diagnostic apparatus 10 according to the first embodiment transmits, similarly to conventional ultrasound diagnostic apparatus explained with reference to FIG. 2, the first ultrasonic pulse 21 to each of the transmission scan line (1), the transmission scan line (3), ..., the transmission scan line (n-3), and the transmission scan line (n-1). Moreover, the ultrasound probe 101 transmits the second ultrasonic pulse 22 to each of the transmission scan line (2), the transmission scan line (4), ..., the transmission scan line (n-2), and the transmission scan line (n) .

As described, the ultrasound probe 101 performs transmission of ultrasonic pulses of different polarities to different transmission positions of the subject P or a contrast agent applied to the subject P multiple times. For example, the ultrasound probe 101 is one example of a transmitting unit.

The receiving circuitry 112 of the ultrasound diagnostic apparatus 10 performs parallel simultaneous reception. That is, the receiving circuitry 112 receives a reflection wave signal by a reflected waved originating from the first ultrasonic pulse 21 (ultrasonic beam) transmitted by the ultrasound probe 101 that is caused to transmit it to the transmission scan line (1) by the transmitting circuitry 111 as plural reception beams, through the ultrasound probe 101 at the plural reception positions at the same time. For example, the receiving circuitry 112 generates a reflection wave signal of plural reception positions as reflection wave signal (reception signal based on a reflected wave originating from the first ultrasonic pulse 21 transmitted to the transmission scan line (1). Specifically, the receiving circuitry 112 generates eight pieces of reflection wave signals 1_1 to 1_8 (not illustrated) of eight reception scan lines (1) to (8) as the reflection wave signal based on a reflected wave originating from the first ultrasonic pulse 21 transmitted to the transmission scan line (1). The reflection wave signal d_k is a reflection wave signal of the reception scan line (k) based on a reflected wave originating from the ultrasonic pulse transmitted to the transmission scan line (d).

The receiving circuitry 112 generates eight pieces of reflection wave signals d_k based on a reflected wave originating from the first ultrasonic pulse 21 similarly for each of the other transmission scan lines (3), ..., (n-1). That is, the receiving circuitry 112 generates eight pieces of reflection wave signals s_(4s-3) to s_(4s+4) (not illustrated) of eight reception scan lines (4s-3) to (4s+4) as a reflection wave signal based on a reflected wave originating from the first ultrasonic pulse 21 transmitted to the transmission scan line (s). Hereinafter, when explaining without distinguishing these reflection wave signals originating from the first ultrasonic pulse 21, the reflection wave signal originating from the first ultrasonic pulse 21 can be denoted simply as "reflection wave signal s".

As described, the receiving circuitry 112 generates m(=4n) pieces of reflection wave signals s.

Moreover, the receiving circuitry 112 generates reflection wave signals of plural reception positions, for example, as a reflection wave signal based on a reflected wave originating from the second ultrasonic pulse 22 transmitted to the transmission scan line (2). Specifically, the receiving circuitry 112 generates eight pieces of reflection wave signals 2_5 to 2_12 (not illustrated) of eight reception scan lines (5) to (12) as the reflection wave signal based on a reflected wave originating from the second ultrasonic pulse 22 transmitted to the transmission scan line (2) .

The receiving circuitry 112 generates eight pieces of reflection wave signals d_k based on a reflected wave originating from the second ultrasonic pulse 22 similarly for each of other transmission scan lines (4), ..., (n) also. That is, the receiving circuitry 112 generates eight pieces of reflection wave signals w_(4w-3) to w_(4w+4) (not illustrated) of eight reception scan lines (4w-3) to (4w+4) as a reflection wave signal based on a reflected wave originating from the second ultrasonic pulse 22 transmitted to the transmission scan line (w). Hereinafter, when explaining without distinguishing these reflection wave signals originating from the second ultrasonic pulse 22, the reflection wave signal originating from the second ultrasonic pulse 22 can be denoted simply as "reflection wave signal w".

As described, the receiving circuitry 112 generates m(=4n) pieces of reflection wave signals w.

The beam former 120 of the ultrasound diagnostic apparatus 10 generates m pieces of reflection wave data 30_1 to 30_m from m pieces of the reflection wave signals s generated by the receiving circuitry 112 by using transmission aperture synthesis using a virtual sound source (transmission aperture synthesis by virtual sound source) as illustrated in FIG. 3. That is, the beam former 120 performs the transmission aperture synthesis using a virtual sound source with respect to m pieces of reflection wave signals s generated by the receiving circuitry 112, to generate m pieces of the reflection wave data 30_1 to 30_m. The reflection wave data 30_k is reflection wave data of the reception scan line (k). The m pieces of the reflection wave data 30_1 to 30_m are reflection wave data that is used when the B-mode image data of one frame is generated, and is reflection wave data originating from the first ultrasonic pulse 21.

The aperture synthesis using a virtual sound source is also called virtual sound source method, and is a publicly known technique. For example, the virtual sound source method is a technique described in Japanese Patent No. 3401199 (Patent Literature 1), JP-A-2009-240700 (Patent Literature 5), Japanese Patent No. 6014643 (Patent Literature 6), and the like. In the virtual sound source method, a transmission focus point (transmission convergence point) is the virtual sound source. In the virtual sound source method, a sound field is to be a field as if transmission focus is applied at points other than the transmission focus point also.

Moreover, the beam former 120 generates m pieces of reflection wave signals 31_1 to 31_m from m pieces of the reflection wave signals w generated by the receiving circuitry 112 by using transmission aperture synthesis using a virtual sound source as illustrated in FIG. 3. That is, the beam former 120 performs the transmission aperture synthesis using a virtual sound source with respect to m pieces of reflection wave signals w generated by the receiving circuitry 112, to generate m pieces of the reflection wave data 31_1 to 31_m. The reflection wave data 31_k is reflection wave data of the reception scan line (k). The m pieces of the reflection wave data 31_1 to 31_m are reflection wave data that is used when the B-mode image data of one frame is generated, and is reflection wave data originating from the second ultrasonic pulse 22.

That is, the beam former 120 performs reception beam forming with respect to plural reception signals acquired by transmission of plural ultrasonic pulses of the same polarity because the virtual sound source is used. For example, the beam former 120 is one example of a beam forming processing unit.

Furthermore, the beam former 120 corrects a misalignment caused because transmission positions to which ultrasonic waves of different polarities are transmitted differ, in the reception beam forming processing. The misalignment herein is a misalignment of a position of a non-linear signal acquired by pulse inversion and a transmission position of the first ultrasonic pulse 21 transmitted to acquire his non-linear signal, and a misalignment of a position of a non-linear signal acquired by pulse inversion and a transmission position of the second ultrasonic pulse 22 transmitted to acquire this non-linear signal. This misalignment varies depending on a depth also. This misalignment is also called misregistration. Moreover, the beam former 120 corrects such a misalignment by the virtual sound source method using plural reception signals acquired by transmission of plural ultrasonic pulses.

The signal processing circuitry 130 of the ultrasound diagnostic apparatus 10 adds up two pieces of the reflection wave data 30_k, 31_k at the same reception position. That is, the signal processing circuitry 130 generates reflection wave data 35_k (THI signal 35_k) in which a fundamental wave component is suppressed and a non-linear component is enhanced by adding the reflection wave data 31_k to the reflection wave data 30_k as illustrated in FIG. 3. As described, the signal processing circuitry 130 generates m pieces of the reflection wave data 35_1 to 35_m. The reflection wave data 35_k is reflection wave data of the reception scan line (k). The m pieces of the reflection wave data 35_1 to 35_m are reflection wave data that is used when B-mode image data of one frame is generated.

As described, the signal processing circuitry 130 extracts the reflection wave data 35_k by adding up the reflection wave data 30_k and 31_k of the same reception position after the reception beam forming. The reflection wave data 35_k is a non-linear signal. Moreover, the function of extracting the reflection wave data 35_k of the signal processing circuitry 130 is one example of an extracting unit.

The signal processing circuitry 130 generates B-mode data of one frame by using m pieces of the reflection wave data 35_1 to 35_m. The image generating circuitry 140 generates one frame of the B-mode image data from one frame of the B-mode data. The control circuitry 170 causes the display 103 to display a B-mode image based on the generated B-mod image data. The ultrasound diagnostic apparatus 10 repeats the processing as described above, and causes the display 103 to display B-mode images sequentially, to thereby display a B-mode moving image on the display 103.

The m pieces of the reflection wave data 30_1 to 30_m and m pieces of the reflection wave data 31_1 to 31_m are data generated by the virtual sound source method. Therefore, even when a transmission position to which the first ultrasonic pulse 21 is transmitted and a transmission position to which the second ultrasonic pulse 22 is transmitted differ, data without misalignment can be acquired, and reflection wave data in which a fundamental wave component is suppressed and that only includes a non-linear signal cam be acquired. Therefore, according to the ultrasound diagnostic apparatus 10 according to the first embodiment, the image quality can be improved.

Moreover, the ultrasound diagnostic apparatus 10 performs parallel simultaneous reception, decreasing the density of transmission scan lines as described above. Therefore, according to the ultrasound diagnostic apparatus 10 according to the first embodiment, reduction of the frame rate can be suppressed.

From the above, according to the ultrasound diagnostic apparatus 10 according to the first embodiment, the image quality of B-mode image data can be improved while suppressing reduction of the frame rate.

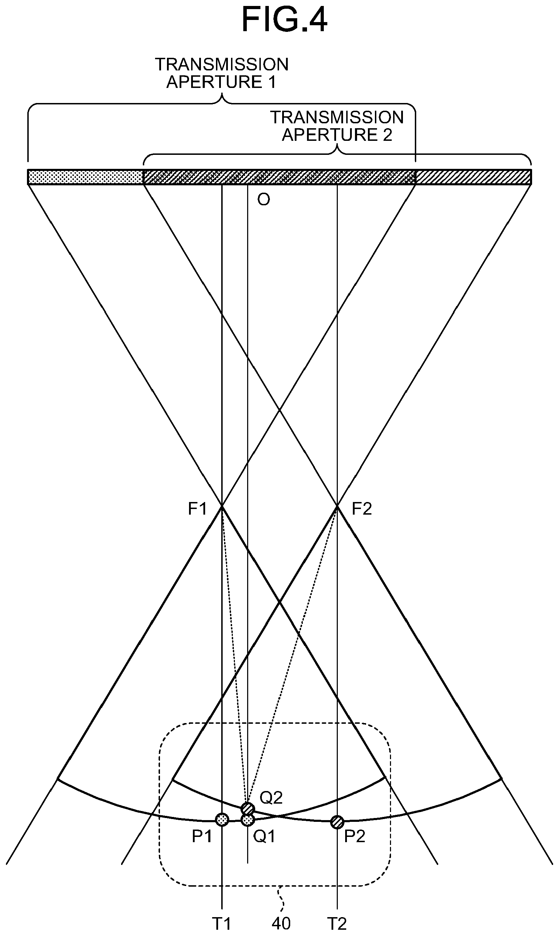

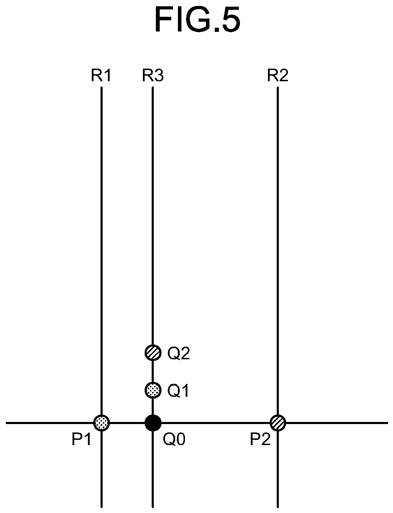

One example of a problem of a conventional ultrasound diagnostic apparatus will be explained, referring to FIG. 4 and FIG. 5. FIG. 4 is a diagram for explaining one example of a problem of the conventional ultrasound diagnostic apparatus. FIG. 5 is an enlarged view of an area 40 in FIG. 4.

As illustrated in FIG. 4, a transmission beam transmitted from a transmission aperture 1 converges at a transmission focus point F1 on a transmission scan line T1. Meanwhile, in a depth direction, a shallow point that is at a shallower position and a deep point that is at a deeper position relative to the transmission focus point F1, the transmission beam spreads. However, in the reception beam forming, a transmission wave front is assumed to propagate as a plane wave. Therefore, at the transmission aperture 1 of the conventional ultrasound diagnostic apparatus, reflection wave signal (reflection wave data) generated by performing the reception beam forming with respect to a position Q0 on a reception scan line R3 illustrated in FIG. 5 is to actually be a reflection wave signal (reflection wave data) of a position Q1 on the reception scan line R3 shifted toward the shallow point relative to the position Q0.

Similarly, although a transmission beam transmitted from a transmission aperture 2 converges at a focus point F2 on a transmission scan line T2, the transmission beam spreads at a shallow point that is at a shallower position and a deep point that is at a deeper position relative to the transmission focus point F2 in a depth direction. Therefore, at the transmission aperture 2 of the conventional ultrasound diagnostic apparatus, a reflection wave signal (reflection wave data) generated by performing the reception beam forming with respect to a position Q0 on the reception scan line R3 illustrated in FIG. 5 is to actually be a reflection wave signal of a position Q2 on the reception scan line R3 shifted toward a shallow side relative to the position Q0. Therefore, the conventional ultrasound diagnostic apparatus that performs pulse inversion of a conventional technique generates a reflection wave signal of the position Q0 by adding the reflection wave signal of the position Q2 to the reflection wave signal of the position Q1. Because the reflection wave signal of the position Q0 thus generated is generated by addition of signals of positions different from the position Q0, a fundamental component remains. Therefore, it is difficult for the conventional ultrasound diagnostic apparatus to extract only a non-linear signal properly.

To solve this problem, the ultrasound diagnostic apparatus 10 according to the first embodiment uses the virtual sound source method as described above. The position Q0 at the transmission aperture 1 is at a position shallower by F1Q0-F1P1 relative to a position P1 on the transmission scan line T1. F1Q0 indicates a distance between the transmission focus point F1 and the position Q0. F1P1 indicates a distance between the transmission focus point F1 and the position P1. Because this is the same as an ultrasonic wave propagates from a point sound source, which is virtually present at the transmission focus point F1, this is called virtual sound source method described above. Specifically, in the reception beam forming of a point of the conventional position Q0, a value acquired by adding OQ0 as a transmission distance (OQ0 indicates a distance between a point O and the position Q0) and a distance from each channel to the position Q0 as a reception distance is used. In contrast, in the virtual sound source method, a signal of a distance acquired by adding F1Q0-F1P1 to the sum of those is used for the transmission aperture 1. In the virtual sound source method, a signal of a distance acquired by adding F2Q0-F2P2 to the sum of those is used for the transmission aperture 2. F2Q0 indicates a distance between the transmission focus point F2 and the position Q0. F2P2 indicates a distance between the transmission focus point F2 and the position P2. Because this is the same as synthesizing the transmission aperture 1 and the transmission aperture 2, this is one kind of transmission aperture synthesis. This method can be expanded to be performed on all transmission scan lines (all transmission apertures). It is noted that because this method is effective only when an effective range of a transmission beam is present at a reception position, the number of reflection wave signals (reflection wave data) of each transmission scanning that can be added is few near a transmission focus, and the number of reflection wave signals (reflection wave data) of each transmission scanning that can be added increases with distance from the transmission focus point. That is, the number of pieces of data of different transmission positions that can be added is few near the transmission focus, and the number of pieces of data of different transmission positions that can be added increases with distance from the transmission focus point.

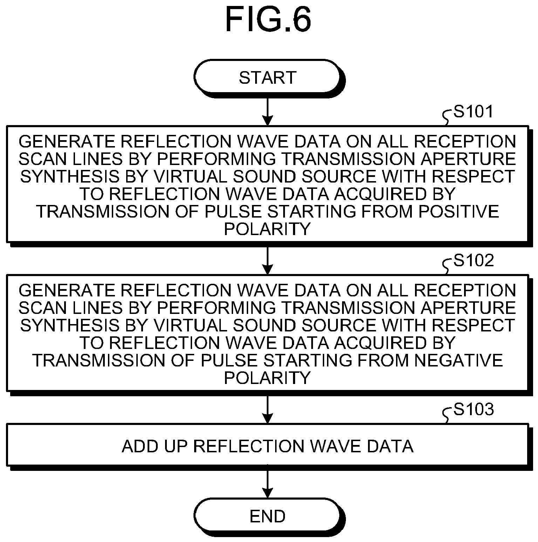

FIG. 6 is a flowchart illustrating a flow of one example of processing performed by the ultrasound diagnostic apparatus 10 according to the first embodiment. The processing illustrated in FIG. 6 is processing of generating reflection wave data in which a fundamental wave component is suppressed.

As illustrated in FIG. 6, the beam former 120 performs the transmission aperture synthesis using a virtual sound source with respect to m pieces of the reflection wave signals s generated by the receiving circuitry 112, to generate m pieces of the reflection wave data 30_1 to 30_m (step S101).

The beam former 120 performs the transmission aperture synthesis by a virtual sound source with respect to m pieces of the reflection wave signals w generated by the receiving circuitry 112, to generate m pieces of the reflection wave data 31_1 to 31_m (step S102).

The signal processing circuitry 130 adds the reflection wave data 31_k to the reflection wave data 30_k, and thereby generates the reflection wave data 35_k (THI signal 35_k) in which a fundamental wave component is suppressed and a non-linear component is enhanced (step S103), and the processing illustrated in FIG. 6 is ended. As described, the signal processing circuitry 130 generates m pieces of the reflection wave data 35_1 to 35_m.

The ultrasound diagnostic apparatus 10 according to the first embodiment has been explained above. According to the ultrasound diagnostic apparatus 10 according to the first embodiment, the image quality of B-mode image data can be improved while suppressing reduction of the frame rate.

### Modification of First Embodiment

The ultrasound diagnostic apparatus 10 according to a modification of the first embodiment will be explained. For example, in the modification of the first embodiment, the misalignment described above may be given by measurement in advance. Moreover, the misalignment described above may be acquired by calculation with simulation (for example, sound field simulation) by the beam former 120. In the modification of the first embodiment, the beam former 120 corrects a misalignment acquired by measurement or simulation by using plural reception signals acquired by transmission of plural ultrasonic pulses, similarly to the first embodiment.

The ultrasound diagnostic apparatus 10 according to the modification of the first embodiment has been explained above. According to the ultrasound diagnostic apparatus 10 according to the modification of the first embodiment, a similar effect to that of the ultrasound diagnostic apparatus 10 according to the first embodiment can be obtained.

A term "processor" used in the above explanation signifies a circuit, such as a central processing unit (CPU), a graphical processing unit (GPU), an application specific integrated circuit (ASIC), a programmable logic device (for example, simple programmable logic device (SPLD), complex programmable logic device (CPLD)), and a field programmable gate array (FPGA). The processor implements a function by reading and executing a program stored in the storage circuitry 160. Moreover, instead of storing a program in the storage circuitry 160, it may be configured to directly install a program in a circuit of the processor. In this case, the processor reads and executes the program installed in the circuit, to implement the function. The respective processors of the present embodiment are not limited to be configured as a single circuitry for each processor, but may be configured by combining plural pieces of independent circuitry as one processor, to implement its function. Furthermore, it may be configured to implement its function by integrating the plural pieces of circuitry in FIG. 1 into one processor. For example, the beam former 120 and the signal processing circuitry 130 may be integrated, and be implemented by a single piece of processing circuitry. This processing circuitry is implemented by a processor.

The control program may be provided being stored in a non-transitory computer-readable storage medium that can be read by a computer, such as a compact disk (CD)-ROM, a flexible disk (FD), a compact disk recordable (CD-R), a digital versatile disk (DVD), in a file of a format that can be installed in or executed by a computer. Moreover, this control program may be stored in a computer connected to a network, such as the Internet, and may be provided or distributed by being downloaded through the network. For example, this control program is constituted of modules including the respective processing functions described above. As actual hardware, the program is read from the storage medium, such as a ROM, to be executed by a processor, and the respective modules are loaded on a main storage device, and are generated on the main storage device.

According to at least one of the embodiment and the modification, the image quality of B-mode image data can be improved while suppressing reduction of the frame rate.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as defined by the appended claims.

## Claims

1. An ultrasound diagnostic apparatus comprising:
a transmitting unit (101) configured to perform transmission of ultrasonic pulses of different polarities to different transmission positions of a subject for a plurality of times;
a beam-forming processing unit (120) configured to perform, for each of the different polarities, reception beam-forming processing with respect to a plurality of reception signals acquired by transmission of a plurality of ultrasonic pulses of an identical polarity; and
an extracting unit (130) configured to extract a non-linear signal by adding up reception signals of the different polarities at an identical reception position, the reception signals subjected to reception beam forming.

2. The ultrasound diagnostic apparatus according to claim 1, wherein
the beam-forming processing unit (120) is configured to correct a misalignment caused by transmission positions being different, the transmission positions to which the ultrasonic waves of different polarities are transmitted, by the reception beam-forming processing.

3. The ultrasound diagnostic apparatus according to claim 2, wherein
the beam-forming processing unit (120) is configured to correct the misalignment by a virtual sound source method by using the reception signals acquired by transmission of the ultrasonic pulses.

4. The ultrasound diagnostic apparatus according to claim 2, wherein
the beam-forming processing unit (120) is configured to correct the misalignment that is acquired by any one of measurement and simulation by using the reception signals acquired by transmission of the ultrasonic pulses.

5. The ultrasound diagnostic apparatus according to any preceding claim, wherein
the beam-forming processing unit (120) is configured to perform, for each of the different polarities, the reception beam-forming processing with respect to the plurality of the reception signals at once.

6. The ultrasound diagnostic apparatus according to any of claims 1 to 4, wherein
the transmitting unit (101) is configured to perform the transmission of the ultrasonic pulses of two polarities that differ from each other to the transmission positions for the plurality of times,
the beam-forming processing unit (120) is configured to perform, for each of two polarities, the reception beam-forming processing with respect to the plurality of the reception signals, and
the extracting unit (130) is configured to extract the non-linear signal by adding up the reception signals of two polarities at the identical reception position, the reception signals subjected to the reception beam forming.

7. An extraction method comprising:
performing transmission of ultrasonic pulses of different polarities to different transmission positions of a subject for a plurality of times;
performing, for each of the different polarities, reception beam-forming processing with respect to a plurality of reception signals acquired by transmission of a plurality of ultrasonic pulses of an identical polarity; and
extracting a non-linear signal by adding up reception signals of the different polarities at an identical reception position, the reception signals subjected to reception beam forming.

8. The extraction method according to claim 7, wherein
the performing the reception beam-forming processing comprises correcting a misalignment caused by transmission positions being different, the transmission positions to which the ultrasonic waves of different polarities are transmitted, by the reception beam-forming processing.

9. The extraction method according to claim 8, wherein
the performing the reception beam-forming processing comprises correcting the misalignment by a virtual sound source method by using the reception signals acquired by transmission of the ultrasonic pulses.

10. The extraction method according to claim 8, wherein
the performing the reception beam-forming processing comprises correcting the misalignment that is acquired by any one of measurement and simulation by using the reception signals acquired by transmission of the ultrasonic pulses.

11. The extraction method according to any of claims 7 to 10, wherein
the performing the reception beam-forming processing comprises performing, for each of the different polarities, the reception beam-forming processing with respect to the plurality of the reception signals at once.

12. The extraction method according to any of claims 7 to 10, wherein
the performing the transmission of the ultrasonic pulses comprises performing the transmission of the ultrasonic pulses of two polarities that differ from each other to the transmission positions for the plurality of times,
the performing the reception beam-forming processing comprises performing, for each of two polarities, the reception beam-forming processing with respect to the plurality of the reception signals, and
the extracting the non-linear signal comprises extracting the non-linear signal by adding up the reception signals of two polarities at the identical reception position, the reception signals subjected to the reception beam forming.
